# EUROPEAN PATENT APPLICATION

(11) **EP 4 488 360 A1**
(43) Date of publication of application: **08.01.2025**
(21) Application number: 23183846.7
(22) Date of filing: 06.07.2023
(51) Int. Cl.: C12N 1/14, B09C 1/10, C02F 3/00, C12P 1/02, C02F 101/36

(54) **DEGRADATION OF FLUOR CONTAINING SUBSTANCES BY BIOCONVERSION**

(71) Applicant: Universiteit Utrecht Holding B.V., 3584 CS Utrecht (NL)
(72) Inventor: Wösten, Herman Abel Bernard, 3705 SN Zeist (NL); van Brenk, Brigit, 2408 SJ Alphen aan den Rijn (NL)
(74) Representative: Nederlandsch Octrooibureau

(57) **Abstract**

The invention relates to the field of degradation of fluor containing substances via bioconversion by edible mushroom forming fungi.

## Description

### Field of the invention

The invention relates to the field of degradation of fluor containing substances by bioconversion.

### Background of the invention

Per- and polyfluoroalkyl substances (PFAS) are highly stable molecules that contain multiple carbon-fluorine bonds (Shahsavari et al., 2021). They are used in various applications including non-stick coatings. Nowadays, PFAS are widespread in the environment. They have been detected in water bodies, household dust, soils and are likely to be present in foods (Shahsavari et al., 2021). Humans are exposed to PFAS through these food and non-food sources (Trudel et al., 2008) and their health can thereby be impacted for instance by affecting kidney, thyroid, or immune function (Shahsavari et al., 2021). Therefore, PFAS have to be removed from the environment.

Several approaches are used or are under study to remove PFAS from the environment (Shahsavari et al., 2021). At the moment, sorption to granular activated carbon (GAC) is the most used way to remove PFAS from drinking water and wastewater. GAC can be reactivated by treatment at high temperature (400-950 °C), thereby degrading the PFAS (DiStefano et al., 2022). However, this method is ineffective for short-chain PFAS removal and the amounts of GAC that are needed are very high (Shahsavari et al., 2021). Surface-Active Foam Fractionation (SAFF) has recently also been shown to be effective in removing PFAS from groundwater (Burns et al., 2021). More than 99.5% of PFOS, PFHxS, and PFOA was removed from contaminated groundwater. SAFF is sustainable since it does not require chemical reagents nor adsorbent media other than air introduced to the foam fractionation vessels. Air bubbles and GAC act both as adsorbents but air bubbles are cheap and do not require disposal after use. As with GAC, longer-chain PFAS species (benefiting from higher adsorption coefficients) are more easy to remove from contaminated water than short-chain species that are typified by lower adsorption coefficient values. Adding an anionic exchange (AIX) resin downstream of SAFF removes all trace detectable PFAS species.

Notably, both GAC and SAFF remove but do not degrade PFAS. An alternative for removal of PFAS is the use of microbes. Microbes can be used to bioaccumulate and biodegrade PFAS. Yet, PFAS is generally recalcitrant to degradation by microbes and the mechanisms of degradation are not known (Wei et al., 2019). Some bacteria (mainly identified as *Pseudomonas* species) can remove 28-67% of PFAS from contaminated soils in a period ranging between 2 and 100 days (Presentato et al., 2020; Yi et al., 2016; Kwon et al., 2014; Huang and Jaffé, 2019). It still has to be assessed whether the PFAS is (fully) degraded and which part is bioaccumulated. Only few studies have examined the ability of fungi to degrade PFAS. The white rot fungus *Phanerochaete chrysosporium* was shown to partly transform 6:2 FTOH, 8:2 FTOH and PFOS (Tseng et al., 2014; Merino 2016). For instance, 50% 6:2 FTOH and 70% 8:2 FTOH were transformed in 28 days into 5:3 polyfluorinated acid (40%), 5:2 FTOH (10%), PFHxA (4%) under laboratory conditions (Tseng et al., 2014; Merino et al., 2016). Highest activity was obtained when the medium was supplemented with lignocellulosic powder, yeast extract, cellulose, and glucose. The fungi *Gloeophyllum trabeum* and *Trametes versicolor* also biotransform 6:2 FTOH but at lower rates (Merino, 2016; Merino et al. 2018). The degradation products included 5:3 acid, 6:2 FTUCA, 5:2 ketone, 5:2 sFTOH, and PFHxA, which show that fungi can degrade 6:2 FTOH to other less fluorinated or more biodegradable metabolites. The articles describing fungal degradation of PFAS did not assess the underlying mechanism but other articles indicate that oxidative enzymes such as peroxidases and laccases are involved (Colosie et al., 2009; Luo et al., 2018; Huang, 2019). Moreover, changing medium composition and the presence of other microbes, as occurs in nature, may stimulate degradation of PFAS (Merino, 2016; Merino et al., 2018).
So, the state of the art methods to remove PFAS from contaminated soils are expensive, impractical for in situ treatment, use high pressure and temperatures, and/or result in toxic waste. Biodegradation by means of microbes has the potential to form the basis of a cost-effective, large scale in situ remediation strategy for PFAS. Literature (see above) has described the use of bioremediation to degrade, sorb and remove certain PFAS from water and soil. The main focus in literature is on bioremediation using bacteria. Only a few studies describe the use of mycoremediation (i.e. remediation with fungi) but success is limited, it involves long incubation times and often only uses one organism. Thus, so far biodegradation is a slow process, which makes it less attractable for industrial processes as waste water treatment

Accordingly, there is a need to improve processes to degrade PFAS.

### Description of the invention

The inventors have established a novel method of degradation of per- and polyfluoroalkyl substances (PFAS) by bioconversion.

Accordingly, there is provided a method for bioconversion of per- and polyfluoroalkyl substances (PFAS) comprising contacting a matter comprising the PFAS with an effective amount of a material colonized by edible mushroom forming fungi, and/or with an effective amount of an extract of a material colonized by edible mushroom forming fungi. Herein, this method is referred to as the "method according to the invention", "the method", or the "method of bioconversion".

The term "bioconversion" is a process known to the person skilled in the art and is herein preferably construed as the conversion of a per- or polyfluoroalkyl substance (PFAS) into another compound by a biological process.

Per- and polyfluoroalkyl substances (PFAS) are known to the person skilled in the art and are herein preferably construed as a group of synthetic organofluorine chemical compounds that have multiple fluorine atoms attached to an alkyl chain. Herein below, a non exhaustive list of PFAS is set forward. The method of bioconversion comprises contacting the matter comprising the PFAS with the effective amount of a material colonized by edible mushroom forming fungi, and/or with the effective amount of an extract of a material colonized by edible mushroom forming fungi. Contacting may be performed using any method known in the art. The materials can e.g. be brought together in a container and mixed once, twice, multiple times or may be mixed continuously.

An effective amount is herein defined as an amount of the material colonized by edible mushroom forming fungi and/or an amount of the extract of a material colonized by edible mushroom forming fungi that mediates at least a measurable conversion of the PFAS in the matter comprising the PFAS.

In the embodiments herein, the matter comprising the PFAS may be any such matter known to the person skilled in the art. The matter may be a liquid or a non-liquid.

In the embodiments herein, the liquid may be any aqueous composition such as, but not limited to, an aqueous liquid such as water. Preferably, the liquid is groundwater, waste water, surface water or sub-surface water.

In the embodiments herein, the non-liquid may be a solid, such as but not limited to, soil or a composition comprising soil. A non-liquid such as soil may be completely dry or may contain some water. Preferably, the non-liquid is soil, earth or a material from a garbage dump.

In the embodiments herein, the PFAS may be any PFAS known to the person skilled in the art and may be grouped as set forward in: www.cdc.gov/exposurereport/pdf/Report_Chemical_List-508.pdf. PFAS typically consist of a fully (per) or partly (poly) fluorinated carbon chain connected to different functional groups. Based on the length of the fluorinated carbon chain, short and long chain PFAS can be distinguished. Long chains refer to:
- perfluorocarboxylic acids (PFCAs) with carbon chain lengths C8 and higher, including perfluorooctanoic acid (PFOA);
- perfluoroalkane sulfonic acids (PFSAs) with carbon chain lengths C6 and higher, including perfluorohexane sulfonic acid (PFHxS) and perfluorooctane sulfonate (PFOS); and
- precursors of these compounds that may be produced or present in products.

A non-exhaustive preferred list of PFAS consists of: Perfluorobutanoic acid (PFBA), Perfluorobutane sulfonic acid (PFBS), Perfluorodecanoic acid (PFDA), Perfluoroheptane sulfonic acid (PFHpS), Perfluorododecanoic acid (PFDoA), Perfluoroheptanoic acid (PFHpA), Perfluorohexane sulfonic acid (PFHxS), Perfluorohexanoic acid (PFHxA), Perfluorononanoic acid (PFNA), Perfluorooctanoic acid (PFOA), n-Perfluorooctanoic acid (n-PFOA), Branched Perfluorooctanoic acid (Sb-PFOA), Perfluorooctane sulfonic acid (PFOS), n-Perfluorooctane sulfonic acid (n-PFOS), Branched Perfluoromethylheptane sulfonic acid (Sm-PFOS), Perfluorooctane sulfonamide (PFOSA or FOSA), Perfluoropentanoic acid (PFPeA), Perfluoroundecanoic acid (PUFA or PFUnDA), 2-(N-Ethyl-perfluorooctane sulfonamido) acetic acid (EtFOSAA), 2-(N-Methyl-perfluorooctane sulfonamido) acetic acid (Me-PFOSA-AcOH, or Me-FOSA-A), 9-Chlorohexadecafluoro-3-oxanonane-1-sulfonic acid (9Cl-PF3ONS), Adona (4,8-dioxa-3H-perfluorononanoate), and GenX (2,3,3,3-tetrafluoro-2-(1,1,2,2,3,3,3-heptafluoropropoxy)-propanoic acid (HFPO-DA)).

Bioconversion of all PFAS listed herein functionally and structurally is within the scope of the claimed invention.

Preferably, in the embodiments herein, the PFAS is selected from the group consisting of: perfluorooctanesulfonate (PFOS), perfluorooctanoate (PFOA), perfluoro-n-butanoic acid (PFBA), perfluoro-n-pentanoic acid (PFPeA), perfluoro-n-hexanoic acid (PFHxA), perfluoro-n-butane sulfonate (PFBS), GenX (2,3,3,3-tetrafluoro-2-(1,1,2,2,3,3,3-heptafluoropropoxy)-propanoic acid, and perfluoro-n-hexane sulfonate (PFHxS).

In the embodiment herein, the material colonized by edible mushroom forming fungi may be spent mushroom substrate (SMS) and/or casing layer and the extract of the material colonized by edible mushroom forming fungi may be tea extracted from SMS and/or from casing layer.

Accordingly, in the embodiments herein, the material colonized by edible mushroom forming fungi may be spent mushroom substrate (SMS), herein also depicted as spent mushroom compost, SMS compost, or spent mushroom substrate compost. The material colonized by edible mushroom forming fungi may be casing layer. The material colonized by edible mushroom forming fungi may be spent mushroom substrate (SMS) and casing layer. The extract of the material colonized by edible mushroom forming fungi may be tea extracted from spent mushroom substrate (SMS). The extract of the material colonized by edible mushroom forming fungi may be tea extracted from casing layer. The extract of the material colonized by edible mushroom forming fungi may be tea extracted from spent mushroom substrate (SMS) and from casing layer.

The material colonized by edible mushroom forming fungi may be any such material known to the person skilled in the art. Typically, such material comprises an agricultural substrate, which can be any material on or in which a fungus orfungal mycelium can grow, providing needs to the developing fungal mycelium or fungus. The substrate may comprise for instance one or more ingredients of the group consisting of soil, wood, wood chips or sawdust, straw, grain, wheat, manure (for instance straw-bedded horse or poultry manure), coffee grounds, waste or recycled paper, soybean meal, corncobs, nut or seed hulls, cocoa bean hulls, gypsum, cottonseed meal, ammonium nitrate, urea, and brewer's grain. The substrate may also comprise agro-industrial waste, produced from sectors such as the food industry and agriculture. Examples of agro-industrial waste are cellulose, hemicellulose, and lignin. The person skilled in the art knows how to choose the most suited substrate.

Spent mushroom substrate (SMS) is herein construed as the material of the agricultural substrate after fungal mycelium has been grown, optionally when mushrooms have grown and preferably after mushrooms, if present, have been removed from the material. SMS was long-time regarded as a waste stream but can be used for a variety of applications. Grimm and Wösten (2018) summarized such applications including the use to produce high-quality compost (Uzun 2004; Polat et al. 2009), use as substrate for production of other mushrooms (Stamets 1993), to feed animals and to improve their health (Song et al. 2007; Nasehi et al. 2017), to make biofuel production more effectively (Phan and Sabaratnam 2012), to produce materials (Jones et al. 2017; Islam et al. 2017; Appels et al. 2018), and to extract enzymes for industries and bioremediation (Phan and Sabaratnam 2012).

Spent mushroom substrate tea is herein construed as an aqueous extract of spent mushroom substrate. Tea may also be extracted from other material colonized by edible mushroom forming fungi, such as casing layer. Aqueous extract is herein construed as that water is mainly used to extract components from the spent mushroom substrate into a liquid, which is thus an aqueous liquid. The aqueous liquid that is incubated with the spent mushroom substrate may comprise other components in addition to water, such as a salt, a buffering agent and a detergent.

The term "fungal mycelium" or "mycelium" is herein defined as the vegetative part of a fungus, consisting of a network of branched, tubular filaments (hyphae) of fungi. A mycelium may be minute and forming a colony that is too small to see by eye, or it may be extensive. Mycelium allows a fungus to absorb water and nutrients from its environment. Mycelium makes up the thallus, or undifferentiated body, of a typical fungus and the mycelium may be microscopic in size and can develop into visible structures, such as rhizomorphs/cords (long strands of hyphae cemented together), or sclerotia (hard compact masses). A mycelium can form fruiting bodies such as brackets, mushrooms, and puffballs under the right environmental conditions and developmental state of the fungus.

The term "mushroom" is herein defined as the fleshy, spore-bearing fruiting body of a fungus, typically produced above ground on a substrate. A typical example of a "mushroom" is the cultivated white button mushroom, *Agaricus bisporus,* hence the word mushroom is most often applied to those fungi (Basidiomycota, Agaricomycetes) that have a stem (stipe), a cap (pileus), and gills (lamellae, sing. lamella) or pores on the underside of the cap. The word "mushroom" is also used for a wide variety of fungal fruiting bodies that produce sexual spores and that either or not have stems, and the term is used even more generally, to describe both the fleshy fruiting bodies of some Ascomycota and the woody or leathery fruiting bodies of some Basidiomycota. Mushroom forms deviating from the standard morphology usually have more specific names, such as "bracket", "puffball", "stinkhorn", and "morel", and gilled mushrooms themselves are often called "agarics" in reference to their similarity to *Agaricus* or their classification as Agaricales. By extension, the term "mushroom" can also designate the entire fungus when in culture or the thallus of species forming the fruiting bodies called mushrooms, or the species itself. Mushrooms have been widely used as foods and are grown on a commercial scale as edible mushrooms. Certain mushrooms are also used as medicine.

The term "spores" is herein defined as a unit of sexual or asexual fungal reproduction that contain all genetic material of a fungus, thereby allowing the fungus to spread and / or to survive.

The term "casing layer" is herein defined as a layer that is in contact with the substrate layer. It comprises moist material, either organic, inorganic, or a combination of the two, placed on top of the colonized substrate, with the purpose to help induce fruiting and to supply moisture to the substrate and the developing fruiting bodies. The skilled person will know how to select the appropriate casing material. The casing layer can be a nutritious or a non-nutritious layer made from materials such as compost, soil, peat moss, vermiculite, coconut coir, or different mixtures of the previous with various additives. The term "additives" is herein defined as mineral supplements, beneficial to the casing material, that supplies essential minerals for mushroom growth, or to balance the pH of the casing layer. The casing layer can be made with or without fungal mycelium. If the casing layer comprises mycelium, it can be made using the Compost Added Casing (CACing) technique, which can accelerate casing colonization. This technique can involve the application of small amounts of compost fully colonized (spawn-run compost) by the mushroom mycelium to the casing layer.

In the embodiments herein, the edible mushroom is preferably a species *of Agaricus, Pleurotus, Lentinula, Auricularia* and *Flammulina.* Preferably, the species is selected from the group consisting of *Agaricus bisporus, Pleurotus ostreatus, Lentinula edodes, Auricularia auricula-juda,* and *Flammulina velutipes.* A most preferred species is *Agaricus bisporus.*

In the embodiments herein, the material colonized by edible mushroom forming fungi or extract thereof may comprise further fungi and/or prokaryotes. These further fungi and/or prokaryotes may be further fungi and/or prokaryotes that grow together with the mushroom forming fungus in the material. These further fungi and/or prokaryotes may also be added to the material colonized by edible mushroom forming fungi or extract thereof. A preferred fungus to be added is one selected from the group consisting of *Mycothermus thermophilus, Phanerochaete* species such as *Phanerochaete chrysosporium, Gloeophyllum* species, such as *Gloeophyllum trabeum, Trametes* species, such as *Trametes versicolor, Aspergillus* species, such as *Aspergillus niger, Penicillium* species, such as *Penicillium chrysogenum, Fusarium* species, *Paecilomyces* species, and *Hypocrea* species. Preferred prokaryotes are *Mycobacterium* species, such as *Mycobacterium vaccae, Bacillus* species, *Pirrelula* species, *Thermus* species and *Pseudomonas* species, such as *Pseudomonas oleovoransh* and *Pseudomonas fluorescens.*

The material colonized by edible mushroom forming fungi or extract thereof may further comprise microbes promoting directly or indirectly growth of the mushrooms and/or the mycelium in the substrate and/or are microbes preventing disease of the fungal mycelium and/or mushrooms. The microbes may have a beneficial effect on mycelium growth, mushroom growth, stimulate mushroom formation, prevent diseases or growth of pathogens. The skilled person will know what microbes to add to the substrate for promoting growth of mushrooms.

In the embodiments herein, the material colonized by edible mushroom forming fungi or extract thereof may further comprise compounds that can be beneficial for the bioconversion of PFAS. Such compounds may be, but are not limited to, nitrogen-containing molecules, calcium, copper, potassium, magnesium, sodium, iron, manganese, zinc, phosphate, and sulphate, and fungal enzymes such as peroxidases and laccases. Another such compound may be H₂O₂. These compounds may already be present in the material colonized by edible mushroom forming fungi or extract thereof, or may be added before or during the incubation with the matter comprising PFAS. In the embodiments herein, an enzyme may be added to the matter comprising the PFAS, to the material colonized by edible mushroom forming fungi or an extract thereof, and/or to the composition resulting from the contacting of the matter comprising the PFAS with the effective amount of a material colonized by edible mushroom forming fungi, and/or with the effective amount of an extract of a material colonized by edible mushroom forming fungi. Such enzyme preferably is a laccase and/or a peroxidase.

The incubation of the matter comprising the PFAS with the material colonized by edible mushroom forming fungi or an extract thereof may be performed at any suitable temperature, such as between about 4 °C and about 50 °C or between about 8 °C and about 45 °C, such as at about 4 °C, 5 °C, 6 °C, 7 °C, 8 °C, 9 °C, 10 °C, 11 °C, 12 °C, 13 °C, 14 °C, 15 °C, 16 °C, 17 °C, 18 °C, 19 °C, 20 °C, 21 °C, 22 °C, 23 °C, 24 °C, 25 °C, 26 °C, 27 °C, 28 °C, 29 °C, 30 °C, 31 °C, 32 °C, 33 °C, 34 °C, 35 °C, 36 °C, 37 °C, 38 °C, 39 °C, 40 °C, 41 °C, 42 °C, 43 °C, 44 °C, 45 °C, 46 °C, 47 °C, 48 °C, 49 °C, or at about 50 °C. In an embodiment, the incubation is performed at between 4 °C and 50 °C or between 8 °C and 45 °C, such as at 4 °C, 5 °C, 6 °C, 7 °C, 8 °C, 9 °C, 10 °C, 11 °C, 12 °C, 13 °C, 14 °C, 15 °C, 16 °C, 17 °C, 18 °C, 19 °C, 20 °C, 21 °C, 22 °C, 23 °C, 24 °C, 25 °C, 26 °C, 27 °C, 28 °C, 29 °C, 30 °C, 31 °C, 32 °C, 33 °C, 34 °C, 35 °C, 36 °C, 37 °C, 38 °C, 39 °C, 40 °C, 41 °C, 42 °C, 43 °C, 44 °C, 45 °C, 46 °C, 47 °C, 48 °C, 49 °C, or at 50 °C.

In the embodiments herein, preferably at least about 1% of the PFAS is converted, preferably into a less toxic or non-toxic compound. In the embodiments herein, at least about 1% is herein preferably at least about 1%, at least about 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 334%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 6%, 67%, 68%, 69%, 79%, 71%, 72%, 73%, 74%, 75%, 76%, 77%, 78%, 79%, 89%, 81%, 82%, 83%, 84%, 85%, 86%, 87%, 88%, 89%, 90%, 91%, 92%, 93%, 94%, 95%, 96%, 97%, 98%, and most preferably at least about 99%.

In the embodiments herein, preferably at least 1% of the PFAS is converted, preferably into in a less toxic or non-toxic compound. In the embodiments herein, at least 1% is herein preferably at least 1%, at least 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 334%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 6%, 67%, 68%, 69%, 79%, 71%, 72%, 73%, 74%, 75%, 76%, 77%, 78%, 79%, 89%, 81%, 82%, 83%, 84%, 85%, 86%, 87%, 88%, 89%, 90%, 91%, 92%, 93%, 94%, 95%, 96%, 97%, 98%, and most preferably at least 99%.

In the embodiments herein, the at least about 1% or the at least 1% PFAS is preferably converted in about 1 minute, 1 hour, 24 hours, one week, two weeks, three weeks, four weeks, one month, two months, three months, four months, five months, six months, seven months, eight months, nine months, ten months, eleven months, or in about one year.

In the embodiments herein, the at least about 1% or the at least 1% PFAS is preferably converted in 1 minute, 1 hour, 24 hours, one week, two weeks, three weeks, four weeks, one month, two months, three months, four months, five months, six months, seven months, eight months, nine months, ten months, eleven months, or in one year.

In a second aspect, there is provided a composition comprising the matter comprising the PFAS and further comprising the effective amount of a material colonized by edible mushroom forming fungi, and/or further comprising the effective amount of an extract of a material colonized by edible mushroom forming fungi. In this aspect, the features preferably are the features as set forward in the first aspect.

Preferably, the matter is a liquid or a non-liquid.

Preferably, the liquid is groundwater, waste water, surface water or subsurface water.

Preferably, the non-liquid is soil, earth or a material from a garbage dump.

Preferably, the PFAS is selected from the group consisting of: perfluorooctanesulfonate (PFOS), perfluorooctanoate (PFOA), perfluoro-n-butanoic acid (PFBA), perfluoro-n-pentanoic acid (PFPeA), perfluoro-n-hexanoic acid (PFHxA), perfluoro-n-butane sulfonate (PFBS), GenX (2,3,3,3-tetrafluoro-2-(1,1,2,2,3,3,3-heptafluoropropoxy)-propanoic acid, and perfluoro-n-hexane sulfonate (PFHxS).

Preferably, the material colonized by edible mushroom forming fungi is spent mushroom substrate (SMS) and/or casing layer and the extract of the material colonized by edible mushroom forming fungi is tea extracted from spent mushroom substrate (SMS) and/or from casing layer.

Preferably, the material colonized by edible mushroom forming fungi or extract thereof comprises further fungi and/or prokaryotes.

Preferably, the composition further comprises an enzyme, which is added to the composition. Preferably, the enzyme is a laccase and/or a peroxidase.

The composition may further comprise compounds that can be beneficial for the bioconversion of PFAS. Such compounds may be, but are not limited to, nitrogen-containing molecules, calcium, copper, potassium, magnesium, sodium, iron, manganese, zinc, phosphate, and sulphate, and fungal enzymes such as peroxidases and laccases. Another such compound may be H₂O₂. These compounds may already be present in the composition or in the material colonized by edible mushroom forming fungi or extract thereof, or may be added to the composition.

In a third aspect, there is provided for a device comprising the composition as defined in aspect 2 In this aspect, the features preferably are the features as set forward in the first and second aspect. Such device is suitable for performing the method of the first aspect.

Preferably, the device is a container or a column.

In a fourth aspect, there is provided for a composition obtainable by the method of the first aspect.

In this aspect, the features preferably are the features as set forward in the first, second and third aspect.

### Figure legends

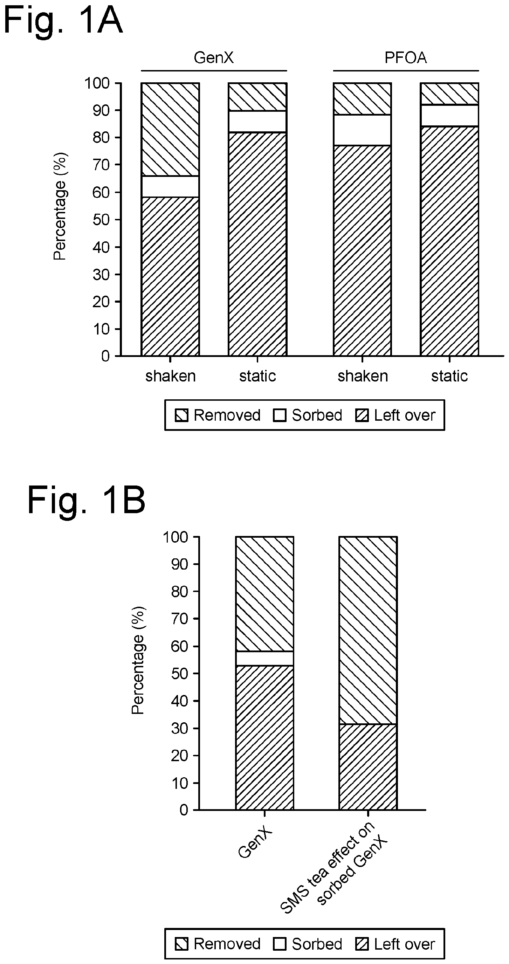

**Figure 1****:** Removal of GenX and PFOA by SMS compost of *Agaricus bisporus* under shaken and static conditions (A) after 24 h when incubated at 21 °C and the effect of overnight SMS tea incubation on sorbed GenX (B).

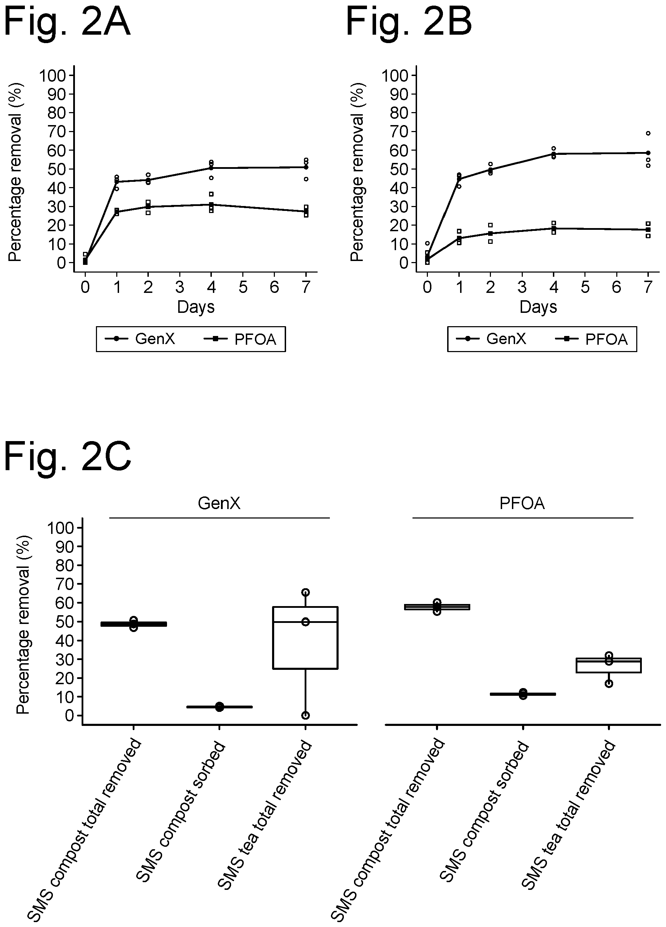

**Figure 2****:** Removal of GenX and PFOA by SMS compost of *Agaricus bisporus* (A) and its tea (B). SMS compost removed PFOA better than the tea, where sorption to SMS compost was minimal (C). GenX was removed the same by SMS and SMS tea and sorption to SMS compost was low.

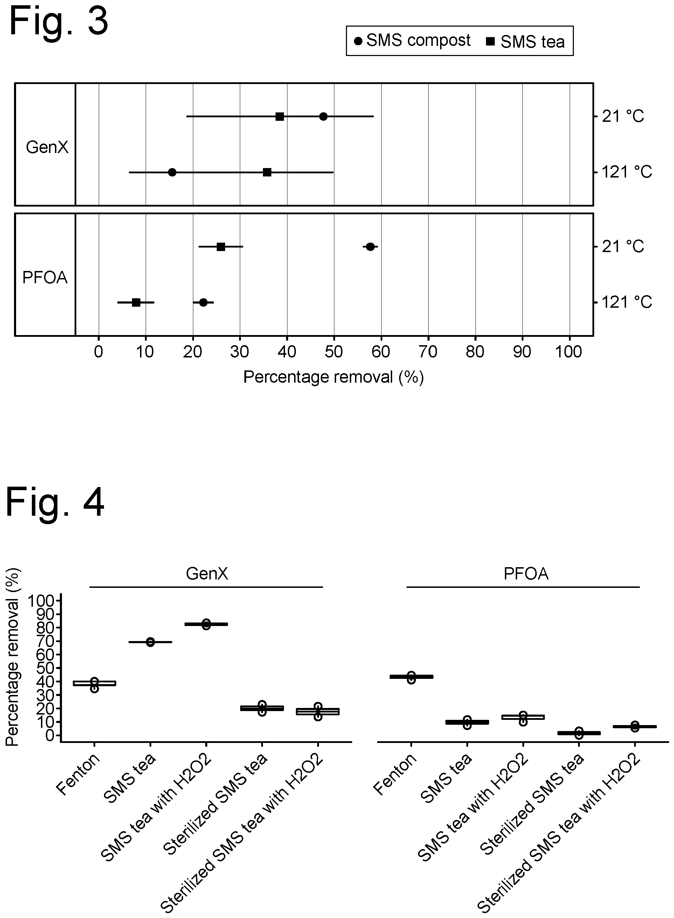

**Figure 3****:** Comparing the removal of GenX and PFOA by non- or heat-treated SMS compost and SMS tea after two days of incubation. Heat-treated SMS compost and SMS tea removed PFOA worse than non-heat treated SMS compost and SMS tea. Heat-treated SMS compost removed GenX worse than non-heat treated SMS compost, while heat treated SMS tea still removed GenX >30%.
**Figure 4****:** GenX and PFOA removal by a heterologous Fenton-like reaction compared to SMS tea and sterilized (heat treated and filtered over 0.22 µM filter) SMS tea with or without addition of extra H₂O₂. Incubation was done for two days. Heterologous Fenton-like reaction cannot explain all removal of GenX by SMS tea.

### Definitions

In this document and in its claims, the verb "to comprise" and its conjugations is used in its non-limiting sense to mean that items following the word are included, but items not specifically mentioned are not excluded. In addition the verb "to consist" may be replaced by "to consist essentially of' meaning that a product or a composition or a nucleic acid molecule or a peptide or polypeptide of a nucleic acid construct or vector or cell as defined herein may comprise additional component(s) than the ones specifically identified; said additional component(s) not altering the unique characteristic of the invention. In addition, reference to an element by the indefinite article "a" or "an" does not exclude the possibility that more than one of the elements is present, unless the context clearly requires that there be one and only one of the elements. The indefinite article "a" or "an" thus usually means "at least one". The word "about" or "approximately" when used in association with a numerical value (e.g. about 10) preferably means that the value may be the given value (of 10) more or less 10% of the value.

All patent and literature references cited in the present specification are hereby incorporated by reference in their entirety.

Unless otherwise indicated each embodiment as described herein may be combined with another embodiment as described herein.

The examples herein are offered for illustrative purposes only, and are not intended to limit the scope of the present invention in any way.

### Further embodiments

Further embodiments of the invention are listed here below.
1. Method for bioconversion of per- and polyfluoroalkyl substances (PFAS) comprising contacting a matter comprising the PFAS with an effective amount of a material colonized by edible mushroom forming fungi, and/or with an effective amount of an extract of a material colonized by edible mushroom forming fungi.
2. The method according to embodiment 1, wherein the matter is a liquid or a non-liquid.
3. The method according to embodiment 2, wherein the liquid is groundwater, waste water, surface water or sub-surface water.
4. The method according to embodiment 2, wherein the non-liquid is soil, earth or a material from a garbage dump.
5. The method according to any one of the preceding embodiments, wherein the PFAS is selected from the group consisting of: perfluorooctanesulfonate (PFOS), perfluorooctanoate (PFOA), perfluoro-n-butanoic acid (PFBA), perfluoro-n-pentanoic acid (PFPeA), perfluoro-n-hexanoic acid (PFHxA), perfluoro-n-butane sulfonate (PFBS), GenX (2,3,3,3-tetrafluoro-2-(1,1,2,2,3,3,3-heptafluoropropoxy)-propanoic acid, and perfluoro-n-hexane sulfonate (PFHxS).
6. The method according to anyone of the preceding embodiments, wherein the material colonized by edible mushroom forming fungi is spent mushroom substrate (SMS) and/or casing layer and the extract of the material colonized by edible mushroom forming fungi is tea extracted from spent mushroom substrate (SMS) and/or from casing layer.
7. The method according to any one of the preceding embodiments, wherein the material colonized by edible mushroom forming fungi or extract thereof comprises further fungi and/or prokaryotes.
8. The method according to any one of the preceding embodiments, wherein an enzyme is added to the matter comprising the PFAS, to the material colonized by edible mushroom forming fungi or extract thereof, and/or to the composition resulting from the contacting of the matter comprising the PFAS with the effective amount of a material colonized by edible mushroom forming fungi, and/or with the effective amount of an extract of a material colonized by edible mushroom forming fungi.
9. The method according to embodiment 8, wherein the enzyme is a laccase and/or a peroxidase.
10. The method according to any one of the preceding embodiments, wherein at least about 1% of the PFAS is converted.
11. A composition comprising the matter comprising the PFAS and further comprising the effective amount of a material colonized by edible mushroom forming fungi, and/or further comprising the effective amount of an extract of a material colonized by edible mushroom forming fungi.
12. The composition according to embodiment 11, wherein the matter is a liquid or a non-liquid.
13. The composition according to embodiment 12, wherein the liquid is groundwater, waste water, surface water or subsurface water.
14. The composition according to embodiment 12, wherein the non-liquid is soil, earth or a material from a garbage dump.
15. The composition according to any one of embodiments 11 to 14, wherein the PFAS is selected from the group consisting of: perfluorooctanesulfonate (PFOS), perfluorooctanoate (PFOA), perfluoro-n-butanoic acid (PFBA), perfluoro-n-pentanoic acid (PFPeA), perfluoro-n-hexanoic acid (PFHxA), perfluoro-n-butane sulfonate (PFBS), GenX (2,3,3,3-tetrafluoro-2-(1,1,2,2,3,3,3-heptafluoropropoxy)-propanoic acid, and perfluoro-n-hexane sulfonate (PFHxS).
16. The composition according to anyone of embodiments 11 to 15, wherein the material colonized by edible mushroom forming fungi is spent mushroom substrate (SMS) and/or casing layer and the extract of the material colonized by edible mushroom forming fungi is tea extracted from spent mushroom substrate (SMS) and/or from casing layer.
17. The composition according to any one of embodiments 11 to 16, wherein the material colonized by edible mushroom forming fungi or extract thereof comprises further fungi and/or prokaryotes.
18. The composition according to any one of embodiments 11 to 17, further comprising an enzyme.
19. The composition according to embodiment 18, wherein the enzyme is a laccase and/or a peroxidase.
20. A device comprising the composition according to any of embodiments 11 to 19.
21. The device according to embodiment 20, wherein the device is a container or a column.
22. A composition obtainable by the method of any one of embodiments 1 to 10.

### Examples

The inventors have, in a non-limiting, exemplary, embodiment, used spent mushroom substrate (SMS) from the white button (*Agaricus bisporus*) industry. The SMS compost contains a microflora (bacteria and fungi; the latter being dominated by *A*. *bisporus*), chemical compounds such as nitrogen-containing molecules, calcium, copper, potassium, magnesium, sodium, iron, manganese, zinc, phosphate, and sulphate, and fungal enzymes such as peroxidases and laccases (see e.g. Gerrits, 1994, Straatsma et al, 2006, Bonnen et al, 1994, Thai et al, 2022). SMS was added to water spiked with 100 - 1000 µg L⁻¹ GenX (also known as HFPO-DA or FRD-903) or PFOA in a volume to volume ratio of 1:20. The mix was incubated under static or shaken (175 rpm) conditions for 24 h at room temperature (21-25 °C). Sorption of PFAS was assessed with a methanol extraction of the SMS after incubation and washing with water. To determine removal over time, the mix was incubated for one week at 100 rpm and room temperature (21-25 °C). Next to mixing SMS with spiked water, removal of PFAS in time was measured by the use of SMS tea. Tea was made by shaking 10 g of SMS with 200 mL water for 1 h at 175 rpm. After filtering through a coffee filter, the tea was spiked with GenX and PFOA to a concentration of 100-1000 µg L⁻¹. Alternatively, the tea was sterilized by heat treatment (121 °C) followed by filtration through a 0.22 µm filter, whereafter the sterilized tea was spiked with GenX and PFOA (100-1000 µg L⁻¹). Samples from the different incubations were filtered using 0.22 µm centrifuge filters (Corning Costar Spin-X) and chromatographic separations were performed with an HPLC e2695 plus Autosampler (Waters Chromatography B.V.). A gradient of solvent A (deionized H₂O (MiliQ) with 0.1% formic acid) and solvent B (methanol (HPLC, Biosolve) with 0.1% formic acid) was used for elution as follows: start with 50:50 (A:B), within 7 minutes linear to 15:85, hold this for 2 minutes, directly back to 50:50, 10 minutes. The gradient was performed at 35 °C at a flow rate of 0.8 mL min⁻¹ using a X Bridge C18 3.5 µm column (100 x 4,6 mm), and a total running time of 10 minutes. GenX and PFOA were detected with a ACQUITY QDa Mass Detector (Waters Chromatopgraphy B.V.) with a negative polarity, capillary potential of 800 V and a source temperature of 450-600 °C.

Results show that SMS removes 10-34% of GenX and 8-12% of PFOA after 1 day when incubated under static or shaken conditions (Fig. 1A). Sorption of the PFAS was in the range of 10% (Fig. 1 and Fig. 2C). Notably, a large part of the sorbed GenX fraction was degraded by an overnight incubation with SMS tea (Fig. 1B). When the SMS PFAS incubation was done during several days, removal can go to 60% of both GenX and PFOA (Fig. 2A; Fig 3). Removal by SMS tea resulted in a removal of 50% of GenX after 7 days, but degradation of PFOA (18%) was less effective (Fig. 2B). SMS and its tea heated at 121 °C for 20 minutes showed less removal of PFOA after two days. The same effect was visible for removal of GenX by SMS. The heat treatment had no effect on the removal of GenX by SMS tea (Fig. 3). This would imply that enzymes are not the main removal mechanism. The fact that metals such as cupper, manganese, and iron are present in SMS compost together with H₂O₂ (Jordan et al., 2008; Medina et al., 2009; Vos, 2017) suggests that a Fenton-like reaction (Hussain et al., 2021) is responsible for this non-enzymatic mechanism. A Fenton-like reaction with CuSO₄ (2.42 mM), MnSO₄ (45.21 mM), FeSO₄ (13.45 mM), and H₂O₂ (0.882 mM) (as present in compost) was compared to treatment with SMS tea and sterilized SMS tea (heattreatment and filtered over a 0.22 µm filter). A total of 40% and 45% of GenX and PFOA was removed by the Fenton-like reaction, respectively (Fig. 4). However, GenX was better removed by SMS tea and SMS tea with addition of H₂O₂. PFOA was less efficiently removed than the Fenton-like reaction, which suggests another mechanism may be involved in the removal of PFOA when compared to GenX.

With the use of SMS, we used a complex substrate which may not only use mycoremediation by A *bisporus* to remove PFAS. The bacterial community, other fungi as well as micro- and macro-nutrients in the compost may be involved as well. This combination of physio-chemical conditions and microbial degradation showed to be effective in the removal of several emerging contaminants, such as pesticides and pharmaceuticals (Lv et al., 2016; Liu et al., 2019). However, instead of using sequential treatments, for example GAC followed by chemical remediation, followed by microbial degradation, the use of SMS provides an all-in-one system. Despite the fact that SMS has been described previously (see e.g. Antón-Herrero et al., 2022; Corral-Bobadilla et al., 2019) to have bioremediation capacity it has never described to remove PFAS.

We conclude that:
- Spent mushroom substrate can remove PFAS from matter by bioconversion.
- Spent mushroom substrate tea can remove PFAS from matter by bioconversion.
- Sorption to the spent mushroom compost is minimal and sorbed PFAS can be removed again with spent mushroom substrate tea.

Accordingly, we conclude that bioconversion of per- and polyfluoroalkyl (PFAS) is possible by contacting a matter comprising the PFAS with an effective amount of a material colonized by edible mushroom forming fungi, and/or with an effective amount of an extract of a material colonized by edible mushroom forming fungi.

### References

Appels FV, Dijksterhuis J, Lukasiewicz CE, Jansen KM, Wösten HAB, Krijgsheld P (2018) Hydrophobin gene deletion and environmental growth conditions impact mechanical properties of mycelium by affecting the density of the material. Sci Rep 8, 4703.
Antón-Herrero R, Garcia-Delgado C, Baena N, Mayans B, Delgado-Moreno L, Eymar E. (2022) Assessment of different spent mushroom substrates to bioremediate soils contaminated with petroleum hydrocarbons. Appl Sci 12, 7720.
Bonnen AM, Anton LH, Orth AB, (1994). Lignin-degrading enzymes of the commercial button mushroom, Agaricus bisporus. Appl Environ Microbiol 60, 960-965
Burns DJ, Stevenson P, Murphy PJC (2021) PFAS removal from groundwaters using Surface-Active Foam Fractionation. Remediation 31, 19-33.
Colosi LM, Pinto RA, Huang Q, Weber WJ Jr (2009) Peroxidase-mediated degradation of perfluorooctanoic acid. Environ Toxicol Chem 28, 264-271.
Corral-Bobadilla M, González-Marcos A, Vergara-González EP, Alba-Elias F (2019) Bioremediation of waste water to remove heavy metals using the spent mushroom substrate of Agaricus bisporus. Water 11, 454.
DiStefano R, Feliciano T, Mimna RA, Redding AM, Matthis J (2022) Thermal destruction of PFAS during full-scale reactivation of PFAS-laden granular activated carbon. Remed J 32:231-238.
Gerrits JPG (1994) Composition, use and legislation of spent mushroom substrate in the Netherlands. Compost Sci Util 2, 24-30
Grimm D, Wösten HAB (2018) Mushroom cultivation in the circular economy. Appl Microbiol Biotechnol 102, 7795-7803.
Huang S, Jaffé PR (2019) Defluorination of perfluorooctanoic acid (PFOA) and perfluorooctane sulfonate (PFOS) by Acidimicrobium sp. strain A6. Environ Sci Technol 53, 11410-11419.
Huang Q (2019) Compositions and methods for perfluoroalkyl acid remediation. (WO2019/169177A1).
Hussain S, Aneggi E, & Goi D, 2021. Catalytic activity of metals in heterogeneous Fenton-like oxidation of wastewater contaminants: a review. Environmental Chemistry Letters, 19, 2405-2424.
Islam MR, Tudryn G, Bucinell R, Schadler L, Picu RC (2017) Morphology and mechanics of fungal mycelium. Sci Rep 7, 13070.
Jones MP, Huynh T, Dekiwadia C, Daver F, John S (2017) Mycelium composites: a review of engineering characteristics and growth kinetics. J Bionanoscience 11, 241-257.
Jordan SN, Mullen GJ, Murphy MC (2008) Composition variability of spent mushroom compost in Ireland. Bioresour Technol 99, 411-418.
Kwon BG, Lim HJ, Na SH, Choi BI, Shin DS, Chung SY (2014) Biodegradation of perfluorooctanesulfonate (PFOS) as an emerging contaminant. Chemosphere 109, 221-225.
Liu X, Guo X, Liu Y, Lu S, Xi B, Zhang J, Wang Z, Bi B (2019) A review on removing antibiotics and antibiotic resistance genes from wastewater by constructed wetlands: Performance and microbial response. Environ Pollut 254, 112996
Luo Q, Liang S, Huang Q (2018). Laccase induced degradation of perfluorooctanoic acid in a soil slurry. J Hazard Mater 359:241-247.
Lv T, Zhang Y, Zhang L, Carvalho PN, Arias CA, Brix H (2016). Removal of the pesticides imazalil and tebuconazole in saturated constructed wetland mesocosms. Water Res 91,126-136
Merino NS (2016) Fungal biotransformation of polyfluoroalkyl substances: identification of growth substrates for favourable biotransformation pathways. PhD Thesis, University of California Los Angeles.
Merino N, Wang M, Ambrocio R, Mak K, O'Connor E, Gao A, Hawley EL, Deeb RA, Tseng LY, Mahendra S (2018) Fungal biotransformation of 6:2 fluorotelomer alcohol. Remed J 28:59-70.
Medina E, Paredes C, Pérez-Murcia MD, Bustamante MA, Moral R (2009) Spent mushroom substrates as component of growing media for germination and growth of horticultural plants. Bioresour Technol 100, 4227-4232.
Nasehi M, Torbatinejad NM, Zerehdaran S, Safaie AR (2017) Effect of solid-state fermentation by oyster mushroom (Pleurotus florida) on nutritive value of some agro by-products. J Appl Anim Res 45, 221-226.
Phan CW, Sabaratnam V (2012) Potential uses of spent mushroom substrate and its associated lignocellulosic enzymes. Appl Microbiol Biotechnol 96, 863-873.
Polat E, Uzun H, Topçuo B, Önal K, Onus AN (2009) Effects of spent mushroom compost on quality and productivity of cucumber ( Cucumis sativus L .) grown in greenhouses. Afr J Biotechnol 8, 176-180.
Presentato A, Lampis S, Vantini A, Manea F, Daprà F, Zuccoli S, Vallini G (2020) On the ability of perfluorohexane sulfonate (PFHxS) bioaccumulation by two Pseudomonas sp. strains isolated from PFAS-contaminated environmental matrices. Microorganisms 8, 92.
Straatsma G, Olijnsma T, Paradi I, Baar J (2006) Minerale voeding van de champignon. https://edepot.wur.nl/297962
Shahsavari E, Rouch D, Khudur LS, Thomas D, Aburto-Medina A, Ball AS (2021) Challenges and current status of the biological treatment of PFAS-contaminated soils. Front Bioeng Biotechnol 8, 602040.
Song YM, Lee SD, Chowdappa R, Kim HY, Jin SK, Kim IS (2007) Effects of fermented oyster mushroom (Pleurotus ostreatus) by-product supplementation on growth performance, blood parameters and meat quality in finishing Berkshire pigs. Animal 1, 301-307.
Stamets P (1993) Growing gourmet and medicinal mushrooms, 3rd ed, Crown Publishing Group, New York.
Thai M, Safianowicz K, Bell TL, Kertesz MA (2022) Dynamics of microbial community and enzyme activities during preparation of Agaricus bisporus compost substrate. ISME Commun 2, 88.
Trudel D, Horowitz L, Wormuth M, Scheringer M, Cousins IT, Hungerbuhler K (2008) Estimating consumer exposure to PFOS and PFOA. Risk Anal 28, 251-269.
Tseng N, Wang N, Szostek B, Mahendra S (2014) Biotransformation of 6:2 fluorotelomer alcohol (6:2 FTOH) by a wood-rotting fungus. Environ Sci Technol 48, 4012-4020.
Uzun I (2004) Use of spent mushroom compost in sustainable fruit production. J Fruit Ornam Plant Res 12, 157-165.
Vos AM (2017) Compost degradation and growth of Agaricus bisporus. PhD Thesis, Utrecht University.
Wei Z, Xu T, Zhao D (2019) Treatment of per- and polyfluoroalkyl substances in landfill leachate: status, chemistry and prospects. Environ Sci Water Res Technol 5, 1814-1835.
Yi LB, Chai LY, Xie Y, Peng QJ, Peng QZ (2016) Isolation, identification, and degradation performance of a PFOA-degrading strain. Genet Mol Res 15, gmr.15028043.

## Claims

1. Method for bioconversion of per- and polyfluoroalkyl substances (PFAS) comprising contacting a matter comprising the PFAS with an effective amount of a material colonized by edible mushroom forming fungi, and/or with an effective amount of an extract of a material colonized by edible mushroom forming fungi.

2. The method according to claim 1, wherein the matter is a liquid or a non-liquid, wherein the liquid preferably is groundwater, waste water, surface water or sub-surface water, and wherein the non-liquid preferably is soil, earth or a material from a garbage dump.

3. The method according to any one of the preceding claims, wherein the PFAS is selected from the group consisting of: perfluorooctanesulfonate (PFOS), perfluorooctanoate (PFOA), perfluoro-n-butanoic acid (PFBA), perfluoro-n-pentanoic acid (PFPeA), perfluoro-n-hexanoic acid (PFHxA), perfluoro-n-butane sulfonate (PFBS), GenX (2,3,3,3-tetrafluoro-2-(1,1,2,2,3,3,3-heptafluoropropoxy)-propanoic acid, and perfluoro-n-hexane sulfonate (PFHxS).

4. The method according to anyone of the preceding claims, wherein the material colonized by edible mushroom forming fungi is spent mushroom substrate (SMS) and/or casing layer and the extract of the material colonized by edible mushroom forming fungi is tea extracted from spent mushroom substrate (SMS) and/or from casing layer.

5. The method according to any one of the preceding claims, wherein the material colonized by edible mushroom forming fungi or extract thereof comprises further fungi and/or prokaryotes.

6. The method according to any one of the preceding claims, wherein an enzyme, preferably a laccase or a peroxidase, is added to the matter comprising the PFAS, to the material colonized by edible mushroom forming fungi or extract thereof, and/or to the composition resulting from the contacting of the matter comprising the PFAS with the effective amount of a material colonized by edible mushroom forming fungi, and/or with the effective amount of an extract of a material colonized by edible mushroom forming fungi.

7. The method according to any one of the preceding claims, wherein at least about 1% of the PFAS is converted.

8. A composition comprising the matter comprising the PFAS and further comprising the effective amount of a material colonized by edible mushroom forming fungi, and/or further comprising the effective amount of an extract of a material colonized by edible mushroom forming fungi.

9. The composition according to claim 8, wherein the matter is a liquid or a non-liquid, wherein the liquid preferably is groundwater, waste water, surface water or subsurface water, and wherein the non-liquid preferably is soil, earth or a material from a garbage dump.

10. The composition according to claim 8 or 9, wherein the PFAS is selected from the group consisting of: perfluorooctanesulfonate (PFOS), perfluorooctanoate (PFOA), perfluoro-n-butanoic acid (PFBA), perfluoro-n-pentanoic acid (PFPeA), perfluoro-n-hexanoic acid (PFHxA), perfluoro-n-butane sulfonate (PFBS), GenX (2,3,3,3-tetrafluoro-2-(1,1,2,2,3,3,3-heptafluoropropoxy)-propanoic acid, and perfluoro-n-hexane sulfonate (PFHxS).

11. The composition according to anyone of claims 8 to 10, wherein the material colonized by edible mushroom forming fungi is spent mushroom substrate (SMS) and/or casing layer and the extract of the material colonized by edible mushroom forming fungi is tea extracted from spent mushroom substrate (SMS) and/or from casing layer.

12. The composition according to any one of claims 8 to 11, wherein the material colonized by edible mushroom forming fungi or extract thereof comprises further fungi and/or prokaryotes.

13. The composition according to any one of claims 8 to 12, further comprising an enzyme.

14. The composition according to claim 13, wherein the enzyme is a laccase and/or a peroxidase.

15. A device comprising the composition according to any of claims 8 to 14, wherein the device preferably is a container or a column.
